# EUROPÄISCHE PATENTSCHRIFT

(11) **EP 1 284 979 B1**
(45) Veröffentlichungstag und Bekanntmachung des Hinweises auf die Patenterteilung: **29.12.2004**
(21) Anmeldenummer: 01940539.8
(22) Anmeldetag: 29.05.2001
(51) Int. Cl.: C07F 7/21, C07F 7/08, A61K 6/08

(54) **PRÄPOLYMERE (METH)ACRYLATE MIT POLYCYCLISCHEN ODER AROMATISCHEN SEGMENTEN**
PREPOLYMERIC (METH)ACRYLATES WITH POLYCYCLIC OR AROMATIC SEGMENTS
(METH)ACRYLATES PREPOLYMERES A SEGMENTS POLYCYCLIQUES OU AROMATIQUES

(30) Priorität: 29.05.2000 DE 10026432
(43) Veröffentlichungstag der Anmeldung: 26.02.2003
(73) Patentinhaber: 3M ESPE AG, 82229 Seefeld (DE)
(72) Erfinder: BISSINGER, Peter, 86911 Diessen (DE); ECKHARDT, Gunther, 06231 Bad Dürrenberg (DE); WEINMANN, Wolfgang, 82205 Gilching (DE)
(74) Vertreter: Brem, Roland
(86) Internationale Anmeldenummer: PCT/EP2001/006100
(87) Internationale Veröffentlichungsnummer: WO 2001/092271

(56) Entgegenhaltungen:
- DE-A- 3 707 908
- DE-A- 19 860 361
- DE-A- 19 860 364
- DE-A- 19 934 407
- US-A- 5 081 164
- GAO, FENG ET AL: "Evaluation of multi-methacrylates copolymerized with methacryl-POSS for potential organic-inorganic hybrid dental restorative materials" POLYM. PREPR. (AM. CHEM. SOC., DIV. POLYM. CHEM.), Bd. 41, Nr. 1, 2000, Seiten 580-581, XP001019703

## Beschreibung

Die Erfindung betrifft multifunktionelle (Meth)acrylate mit polycyclischen oder aromatischen Segmenten sowie daraus hergestellt Massen, insbesondere Dentalmassen.

Bei der restaurativen Zahnheilkunde nimmt der ästhetische Aspekt immer mehr zu. Dem Patienten kann insbesondere im Frontzahnbereich nicht mehr zugemutet werden, eine nicht dem natürlichen Zahn entsprechende Restauration zu erhalten. Schlüssel zur Ästhetik ist die Opazität des Restaurationsmaterials.

Unter Restaurationsmaterialien im Sinne dieser Anmeldung werden insbesondere Zahnfüllmaterialien, Stumpfaufbaumaterialien, Zahnzemente, Zahnlacke sowie zahntechnische Materialien, wie Verblendmaterialien verstanden.

Dentale Restaurationsmaterialien basieren auf härtbaren Monomeren bzw. Monomergemischen. Vorwiegend werden dabei im Stand der Technik kurzkettige Monomere auf der Basis von (Meth)acrylaten verwendet, die zu einem nicht unerheblichen Gesundheitsgefährdungsrisiko durch die Auslösung von nichtpolymerisierten Restmonomeren aus dem Restaurationsmaterial führen. Es hat daher nicht an Versuchen gefehlt, auf andere Monomersysteme auszuweichen.

So beschreibt beispielsweise die Anmeldung DE-198 60 364 härtbare Monomere auf der Basis von cyclischen Siloxan(meth)acrylaten. Diese zeichnen sich aus durch eine hohe (Meth)acrylatfunktionalität, die bei daraus hergestellten härtbaren Massen zu guten physikalischen Eigenschaften führt.

Im Rahmen der Anmeldung DE-198 60 361 werden cyclische Sol-Gelkondensierbare Siloxane beschrieben, die in daraus hergestellten härtbaren Massen zu einem niedrigen Polymerisationsschrumpf und verbesserten physikalischen Eigenschaften führen.

Ganz allgemein fehlt es im Stand der Technik nicht an polymerisierbaren Massen, die sich durch niedrigen Schrumpf und verbesserte physikalische Werte auszeichnen; sehr wohl fehlt es aber an härtbaren Massen, die verbesserte ästhetische Eigenschaften, insbesondere eine Opazität aufweisen, die den visuellen Eindruck natürlicher Zahnsubstanz gewährleistet.

Der vorliegenden Anmeldung liegt die Aufgabe zugrunde, Monomere mit hohem Molekulargewicht zur Verfügung zu stellen, die die Formulierung von Dentalmassen erlauben, die im ausgehärteten Zustand eine geringe Opazität aufweisen, um den visuellen Eindruck natürlicher Zahnsubstanz zu gewährleisten.

Diese Aufgabe wird durch die erfindungsgemäßen Monomere und den daraus formulierten Massen gemäß den Ansprüchen gelöst.

Vorteilhaft an den erfindungsgemäßen Monomeren ist neben der Tatsache, dass sie ein hohes Molgewicht, beispielsweise über 600 g/mol, bevorzugt über 800 g/mol besitzen auch eine hohe (Meth)acrylatfunktionalität, beispielsweise über 2, bevorzugt über 3 bei überraschend niedriger Viskosität. Sie sind daher hervorragend zur Formulierung von leicht applizierbaren Massen geeignet.
Das Molgewicht der erfindungsgemäßen Monomeren kann so hoch sein, bis die Grenze der Fließfähigkeit bei 23°C erreicht wird.

Die aus den Monomeren formulierten Massen besitzen einen niedrigen Polymerisationsschrumpf und sehr hohe mechanische Festigkeit. Somit ist es auch möglich, Massen zu formulieren, ohne übliche niederfunktionelle Monomere auf reiner (Meth)acrylatbasis zu verwenden, was beispielsweise eine Gefährdung des Patienten durch austretende Restmonomere vermindert.

Die aus den Monomeren formulierten Massen weisen insbesondere eine Opazität von 80 bis 90%, bevorzugt 83 bis 87% auf.

Vorteilhaft und überraschend ist ferner, dass die Monomere trotz ihrer hohen Anzahl von Acrylat- bzw. Methacrylatgruppen pro Molekül lagerstabil sind.

Die erfindungsgemäßen Monomere weisen die allgemeinen Formeln (1) oder (2) auf, wobei den Formeln die kovalente Anknüpfung eines Restes E an ein Siloxan-Backbone gemeinsam ist. 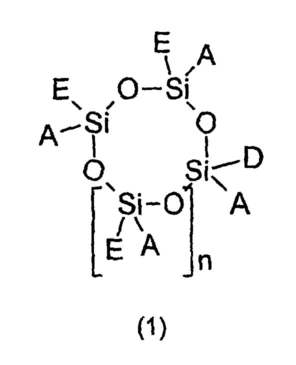 in welcher bedeuten:
- n =: eine ganze Zahl von 0 bis 10, bevorzugt 1 bis 5;
- A =: H oder lineare oder verzweigte C₁- bis C₁₅-Alk(en)yl, bevorzugt Methyl, Ethyl, Propyl, Butyl, Vinyl, Ethinyl, Allyl, oder C₃- bis C₁₅-Cycloalk(en)yl, bevorzugt Cyclopentyl, Cyclohexyl, Cyclopentadienyl, Cyclohexenyl, oder C₆- bis C₁₂-Aryl, bevorzugt Phenyl, Tolyl, Xylyl, oder C₈-C₁₈-Alkaryl, bevorzugt Phenylethylenyl,
wobei aus den genannten Resten jeweils ein oder mehrere C-Atome durch O, C=O, O(C=O), SiR₂ und/oder NR ersetzt sein können, wobei R ein aliphatischer Rest mit 1 bis 7 C-Atomen ist, bei dem ein oder mehrere C-Atome durch O, C=O und/oder O(C=O) ersetzt sein können;
- D =: E oder ein Kohlenwasserstoff-Gerüst, das 2 bis 10, bevorzugt 2 bis 5 Cyclosiloxanreste verknüpft, wobei das Gerüst linear oder verzweigt oder cyclisch oder polycyclisch ist und 2 bis 50, bevorzugt 2 bis 30 C-Atome und zusätzlich 0 bis 30, bevorzugt 0 bis 20 andere Atome aus der Gruppe O, N, S, P, Cl, F, Br, I enthält und an welchem 1 bis 9, vorzugsweise 1 bis 4 der oben definierten Cyclosiloxanreste gemäß Formel (1), abzüglich D, hängen. Bevorzugte Reste D sind: Di(prop-3-yl)ether, Di(prop-3-yl)sulfid, Di(prop-3-yl)amin, Di(prop-3-yl)methylamin, Tri(prop-3-yl)amin, Di(prop-3-yl)harnstoff, Di(prop-3-yl)carbonat, Ethylenglykoldi(prop-3-yl)carbonat, Diethylenglykoldi(prop-3-yl)carbonat, Ethylenglykoldi(prop-3-yl)ether, Diethylenglykoldi(prop-3-yl)ether, 1,2-Propandioldi(prop-3-yl)ether, 1,3-Propandioldi(prop-3-yl)ether, 1,3-Butan-dioldi(prop-3-yl)ether, 1,4-Butandioldi(prop-3-yl)ether, 1,4-Butendioldi(prop-3-yl)ether, 1,4-Butindioldi(prop-3-yl)ether, 1,5-Pentandioldi(prop-3-yl)ether, 1,6-Hexandioldi(prop-3-yl)ether, 1,8-Octandioldi(prop-3-yl)ether, 1,9-Nonandioldi(prop-3-yl)ether, 1,10-Decandioldi(prop-3-yl)ether, 1,12-Dodecandioldi(prop-3-yl)ether, Oxalsäuredi(prop-3-yl)ester, Malonsäuredi(prop-3-yl)ester, Bernsteinsäuredi(prop-3-yl)ester, Adipinsäuredi(prop-3-yl)ether, Sebacinsäuredi(prop-3-yl)ether, 1,2-Ethandiyl, 1,4-Pentadienyl, 1,5-Pentandiyi, 1,5-Hexadienyl, 1,6-Heptadienyl, 1,7-Octadienyl, 1,8-Nonadienyl, 1,9-Decadienyl, 1,11-Dodecadienyl, p-Di(eth-2-yl)benzol, Bis-(4-(prop-3-yl)oxyphenyl)-sulfon, Bis-(4-(prop-3-yl)oxyphenyl)-keton, Bis-(4-(prop-3-yl)oxyphenyl)-methan, 1,1-Bis-(4-(prop-3-yl)oxyphenyl)-ethan, 2,2-Bis-(4-(prop-3-yl)oxyphenyl)-propan, 2,2-Bis-(4-(prop-3-yl)oxyphenyl)-perfluorpropan, 2,2-Bis-(4-(prop-3-yl)oxy-3,5-dibrom-phenyl)-propan, 3,3-Bis-(4-(prop-3-yl)oxyphenyl)-pentan, 4,4-Bis-(4-(prop-3-yl)oxyphenyl)-heptan, 1,1-Bis-(4-(prop-3-yl)oxyphenyl)-cyclopentan, 1,1-Bis-(4-(prop-3-yl)oxyphenyl)-cyclohexan, 1,1-Bis-(4-(prop-3-yl)oxyphenyl)-3,3,5-trimethylcyclohexan, 1,1,1-Tris-(4-(prop-3-yl)oxyphenyl)-ethan, Bis-((prop-3-ylether)oxy)-tricyclo[5.2.1.0^{2.6}]-decan;
- E =: A oder eine polymerisierbare Gruppe entnommen aus der Gruppe G-C[(Q-T-L)ₐ(A)₃₋ₐ] und/oder Q-L und gegebenenfalls zusätzlich X-T-L, wobei bis zu 50%, bevorzugt unter 25% bis 0% der Gruppen E in einem durchschnittlichen Molekül A entsprechen dürfen, mit der Maßgabe, dass bei Molekülen mit nur einem Siloxanring unter 25% bis 0% der Gruppen E in einem durchschnittlichen Molekül A entsprechen dürfen, und mit der Maßgabe, dass mindestens eine Gruppe G-C[(Q-T-L)ₐ(A)₃₋ₐ] und/oder Q-L im Molekül enthalten sein muss;
- G =: lineare, verzweigte oder cyclische C₁- bis C₂₅-, bevorzugt C₁- bis C₂₀-Alk(en)ylen, Arylen, Alkarylen, Arylalkylen, wobei 0 bis 5 C-Atome ersetzt sein können durch einen Vertreter der Gruppe O, S, N-A, C(O), C(O)O, OC(O), C(O)N, NC(O), OC(O)O, NC(O)O, OC(O)N, NC(O)N;
- X =: C₁- bis C₁₀-Alk(en)ylen, bevorzugt Ethylen, Methylethylen, Propylen, Butylen, Hexylen, Ethenylen, Propenylen;
- Q =: ein in der Kette ein aromatisches oder polycyclisches Ringsystem enthaltender Rest, mit 5 bis 20, bevorzugt 6 bis 15 C-Atomen, der zusätzlich unabhängig voneinander 0 bis 5 Heteroatome aus der Gruppe O, N-A, S im Ringsystem aufweist;
- T =: O, N-A oder ein zwei- oder mehrwertiger linearer, verzweigter oder cyclischer Alkohol-, Amin- oder Aminoalkohol-Rest mit 2 bis 10 C-Atomen, bevorzugt Ethandiol-diyl, 1,4,7-Trioxaheptan-diyl, 1,4,7,10-Tetraoxadektan-diyl, 1,4,7,10,13-Pentaoxatridekan-diyl, Glycerin-triyl, Trimethylolpropan-triyl, Pentaerythrit-tetryl;
- L =: eine Acrylat- oder Methacrylatgruppe;
- a =: 2,3. 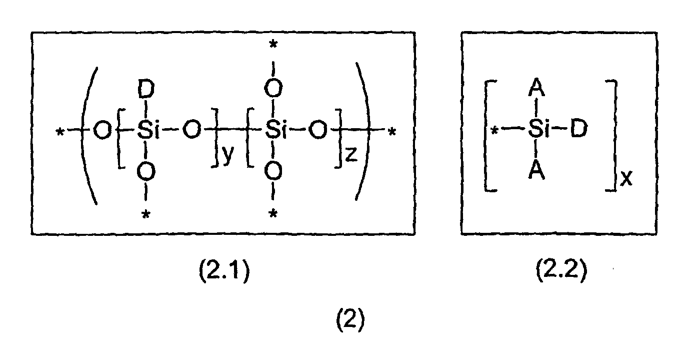 in welcher bedeuten:
- x ≤: 2 + y + 2z;
- y =: 0, 1, 2, 3, 4, 5, 6, 7, 8, bevorzugt 0, 1, 2, 3;
- z =: 0, 1, 2, 3, 4, 5, 6, 7, 8, bevorzugt 0, 1, 2, 3;
A und D haben die obige Bedeutung mit der Maßgabe, dass mindestens 3 der Reste D in einem Molekül die Bedeutung von G-C[(Q-T-L)ₐ(A)₃₋ₐ] oder Q-L oder X-T-L haben müssen, und mit der Maßgabe, dass bei y = z = 0 mindestens 1 Rest D die Bedeutung G-C[(Q-T-L)ₐ(A)₃₋ₐ] hat;
und die Symbole "*" in (2.1) die Anknüpfungspunkte für Fragmente (2.2) über deren Valenz symbolisiert durch ein "*" an das Fragment (2.1) bedeuten.

Jede Auswahl eines über Indizes oder über Mehrfachnennung des Symbols mehrmals genannten Restes im Rahmen dieser Anmeldung ist unabhängig von jeder anderen Auswahl aus der selben Gruppe zu betrachten. Beispielsweise kann die dreifache Nennung eines Restes A in einem Molekül bedeuten, dass die Position von A sowohl durch Methyl- als auch Ethyl- als auch Propyl- im gleichen Molekül ersetzt werden kann.

Verbindungen gemäß Formel (1) sind auf Basis cyclischer Siloxane, wobei ein oder mehrere Siloxan-Ringe pro Molekül vorkommen können.

Verbindungen gemäß Formel (2) sind auf Basis linearer oder verzweigter Siloxane, wobei ein oder mehrere Siloxanreste pro Molekül vorkommen können.

Die Herstellung von Verbindungen der allgemeinen Formeln (1) und (2) geschieht vorzugsweise durch Hydrosilylierung. Hierdurch lassen sich Si-H-funktionelle Cyclosiloxane und verzweigte bzw. lineare Siloxane mit C-C-ungesättigten organischen Gerüsten verknüpfen (B. Marciniec: Comprehensive Handbook on Hydrosilylation, Pergamon Press, 1992). Auf diese Weise lassen sich gewünschte Monomer Strukturen der allgemeinen Formeln darstellen.

Bevorzugte Si-H-funktionelle Silikonkernstücke für die Umsetzung zu erfindungsgemäßen Vertretern der Formel (1) und (2) sind beispielhaft und umfassen ohne Einschränkung: D^{H}₄, D^{H}₅, D^{H}₇, D^{H}₈, ein Gemisch aus D^{H}ₓ, mit x = 4, 5, 6, 7, 8, 9, 10 (Summe dieser Komponenten >90 %), M^{H}₂, M^{H}₄Q, M^{H}₃T, M^{H}₃T^{H}, M^{H}₃T^{Ph}, M^{H}₈Q₈, T^{H}₈ (Silikonnomenklatur gemäß Encyclopedia of Polymer Science and Engineering 2. Ed. Bd. 15 S.206f.; H = hydrido-funktionalisiert, Ph = Phenyl).

Beispielsweise lässt sich 1,3,5,7-Tetramethylcyclotetrasiloxan in einem Lösungsmittel, wie Toluol, unter Einfluss von Edelmetallkatalysatoren, wie (ohne Einschränkung darauf) Speier-Katalysator, Karstedt-Katalysator aber auch Wilkinson-Katalysator, mit vier Mol Vinylbenzylmethacrylat zu einem Vertreter von (1) verknüpfen. Anstelle des Cyclotetrasiloxans lässt sich auch ein kommerziell verfügbares SiH-Cyclengemisch (Petrarch, M8830) (ein Gemisch von D^{H}ₓ, mit x = 4, 5, 6) verwenden. Statt Vinylbenzylmethacrylat können andere ungesättigte Verbindungen, wie Vinylnorbornenolmethacrylat oder Allylether, -ester oder -amide von (Meth)acrylat-funktionellen Bisphenol-A-Derivaten, sowie endocyclisch ungesättigte und andere (Meth)acryl-funktionelle organische Moleküle verwendet werden. Eine weitere erfindungsgemäße Möglichkeit ist, die Cyclosiloxankomponente nicht allein mit aromatischen oder polycyclischen hydrosilylierbaren (Meth)acrylaten umzusetzen, sondern dies im Gemisch mit aliphatischen hydrosilylierbaren (Meth)acrylaten, wie beispielsweise Allyloxyethylmethacrylat oder Glycerindimethacrylat-allylether, zu tun. Erfindungsgemäße Produkte sind dabei jedoch nur diejenigen Anteile, die mindestens eine aromatische oder polycyclische (Meth)acrylatkomponente im Molekül tragen.

Bei der Umsetzung von polyfunktionellen SiH-Cyclosiloxanen mit ebenfalls mehrfach C-C-ungesättigten organischen Gerüsten können durch geeignete Reaktionsführung alle C-C-ungesättigten Funktionen des organischen Gerüstes (mit Ausnahme der polymerisierbaren Doppelbindungen) mit jeweils einem Cyclosiloxanring abgesättigt werden. Durch davon abweichende Wahl der Stöchiometrie oder Reaktionsführung lassen sich jedoch auch vorvernetzte Zwischenprodukte herstellen.
Beide Möglichkeiten sind unabhängig voneinander anwendbar.

Die im folgenden aufgezeigten Strukturen können in an sich bekannter Weise durch Hydrosilylierung von entsprechenden Si-H-Verbindungen, beispielsweise mit Allyloder Vinyl-Verbindungen, erhalten werden. Bei der Hydrosilylierung werden abhängig von den Substraten und dem Katalysator ein Gemisch isomerer Addukte zu unterschiedlichen Anteilen (α- und β-Addukt: siehe Schema (I)) erhalten. In den Formeln ist jeweils nur ein Isomeres gezeigt, gemeint sind jedoch alle Möglichkeiten.

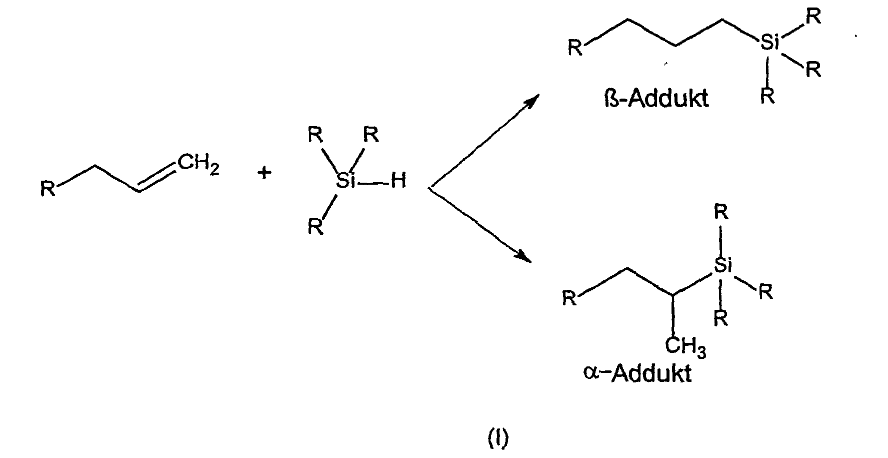

Neben den in den bevorzugten Beispielen gezeigten Methacrylaten sind auch die entsprechenden Acrylate sowie gemischte Typen bevorzugt.

Bevorzugte Vertreter der Formel (1) sind: 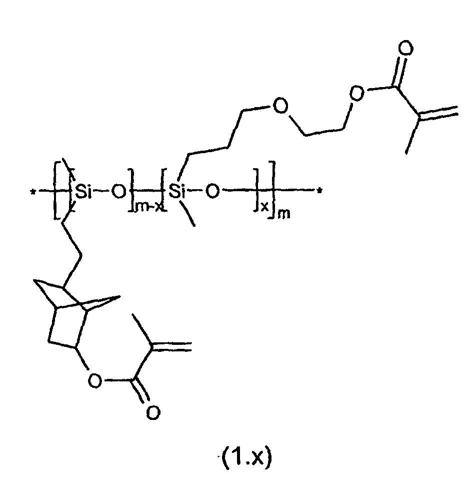 m = 4, 5 oder ein Gemisch aus 4, 5 und 6 (d.h.: n = 0, 1, 2); x = 1 bis m-1, Q = 2(3)-Oxyl-bicyclo[2.2.1]hept-5-(1,2-ethandiyl)-yl; X = 1,2-Ethylen, T = 1-Oxa-1,3-propylen, L = Methacrylat; 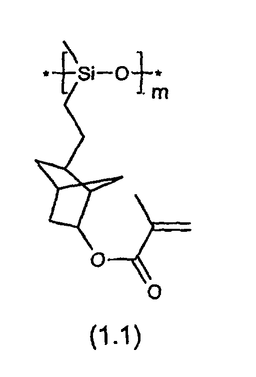 m = 4, 5 oder ein Gemisch aus 4, 5 und 6 (mit n = 0, 1, 2); Q = 2(3)-Oxylbicyclo[2.2.1]hept-5-(1,2-ethandiyl)-yl; L = Methacrylat; 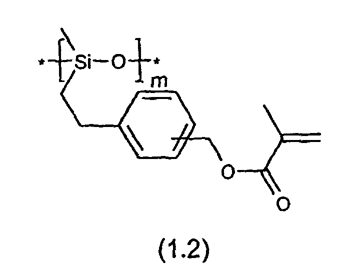 m = 4, 5 oder ein Gemisch aus 4, 5 und 6 (mit n = 0, 1, 2); Q = 1,2-Ethylen-(2-(4-methylenoxyl-1,4(3)-phenylen); L = Methacrylat; 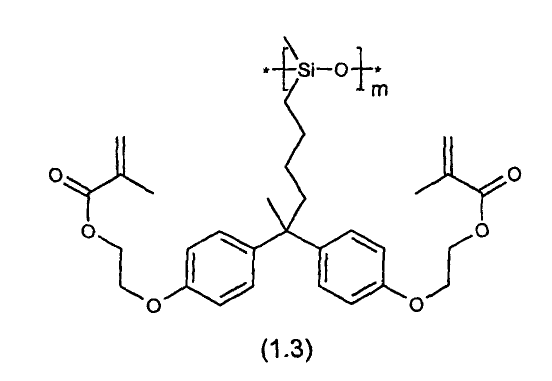 m = 4, 5 oder ein Gemisch aus 4, 5 und 6 (mit n = 0, 1, 2); A = Methyl; D = E; G = 1,4-Butylen, a = 2; Q = 1,4-Phenylen; T = 1,2-Ethandiol-diyl; L = Methacrylat. 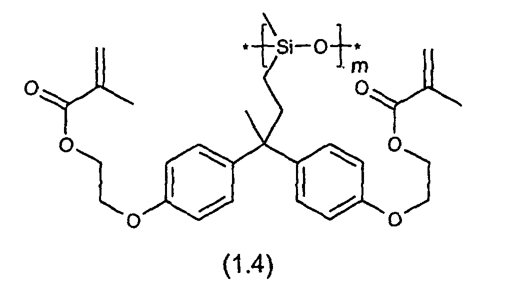 m = 4, 5 oder ein Gemisch aus 4, 5 und 6 (mit n = 0, 1, 2); A = Methyl; D = E; G = 1,2-Ethylen, a = 2; Q = 1,4-Phenylen; T = 1,2-Ethandiol-diyl; L = Methacrylat. 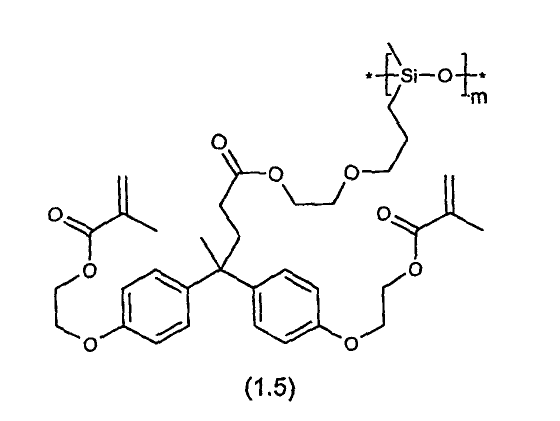 m = 4, 5 oder ein Gemisch aus 4, 5 und 6 (mit n = 0, 1, 2); A = Methyl; D = E; G = 4,7-Dioxa-8-oxo-1,10-decylen, a = 2; Q = 1,4-Phenylen; T = 1,2-Ethandiol-diyl; L = Methacrylat. 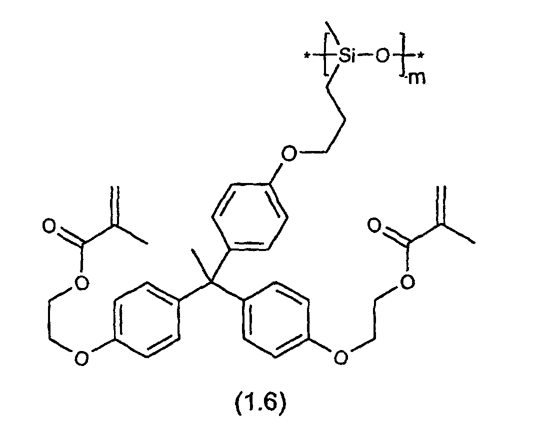 m = 4, 5 oder ein Gemisch aus 4, 5 und 6 (mit n = 0, 1, 2); A = Methyl; D = E; G = 4-Oxa-1,4-butylen-4-(1,4-phenylen, a = 2; Q = 1,4-Phenylen; T = 1,2-Ethandiol-diyl; L = Methacrylat. 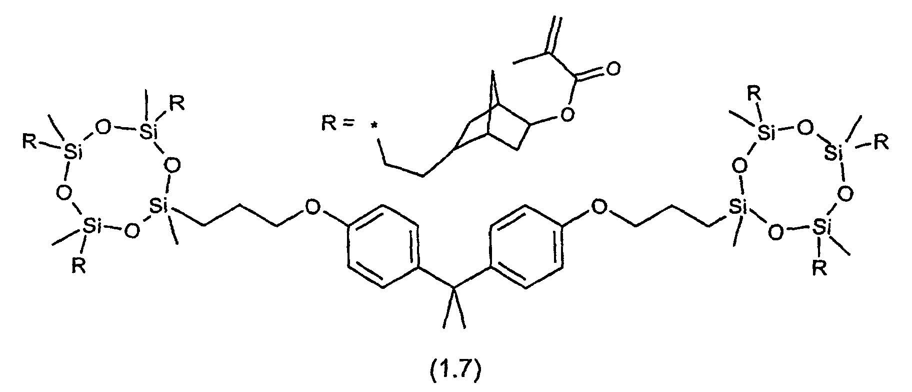 m = 4 (mit n = 0); A = Methyl; D = E, Bis-[2,2-propandiyl-(4-(1,3-propylen-oxy)-1-phenylen); Q = 2(3)-Oxyl-bicyclo[2.2.1]hept-5-(1,2-ethandiyl)-yl; L = Methacrylat; 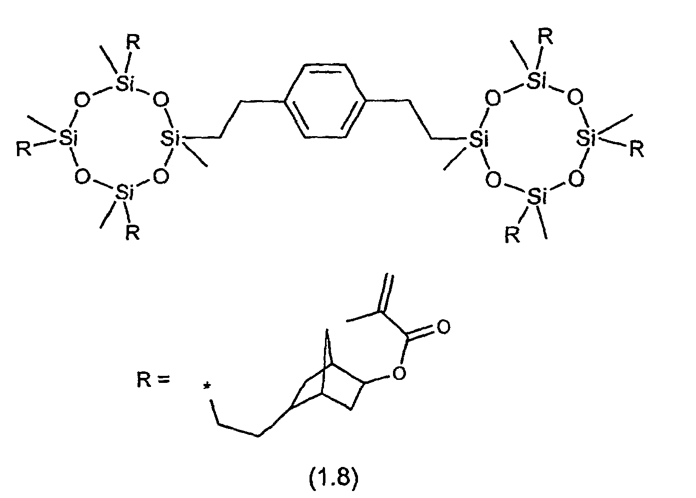 m = 4 (mit n = 0); A = Methyl; D = E, Bis-[1,4-(1,2-ethandiyl)-phenylen); Q = 2(3)-Oxyl-bicyclo[2.2.1]hept-5-(1,2-ethandiyl)-yl; L = Methacrylat; 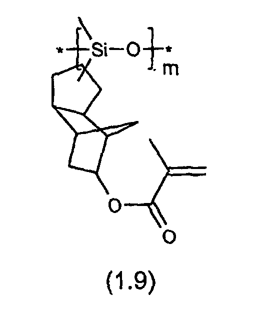 m = 4, 5 oder ein Gemisch aus 4, 5 und 6 (mit n = 0, 1, 2); A = Methyl; D = E; Q = 2(3)-Oxyl-tricyclo[5.2.1.0^{2,6}]dekan-6(7)-yl; L = Methacrylat;

Bevorzugte Vertreter der Formel (2) sind: 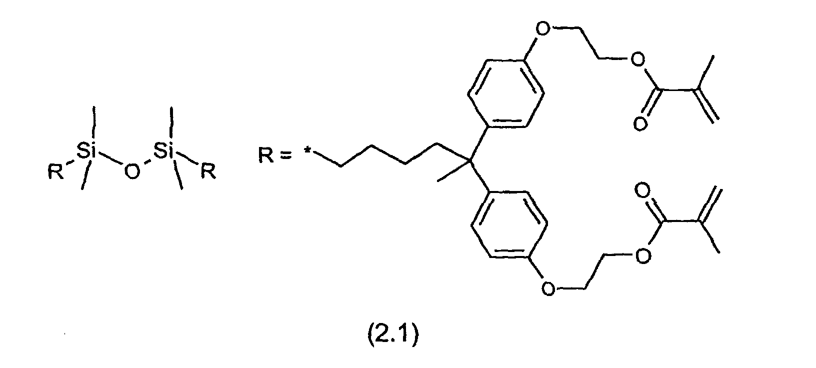 x = 2; y = 0; z = 0; K = Methyl, G-C[(Q-T-L)ₐA₃₋ₐ]; G = 1,4-Butylen; A = Methyl; a = 2; Q = 1,4-Phenylen; T = 1,2-Ethandiol-diyl; L = Methacrylat. 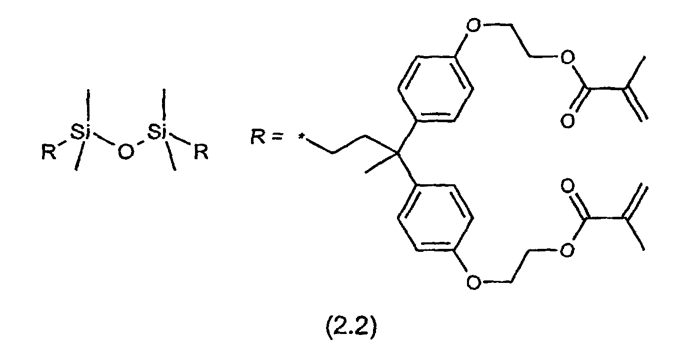 x = 2; y = 0; z = 0; K = Methyl, G-C[(Q-T-L)ₐA₃₋ₐ]; G = 1,2-Ethylen; A = Methyl; a = 2; Q = 1,4-Phenylen; T = 1,2-Ethandiol-diyl; L = Methacrylat. 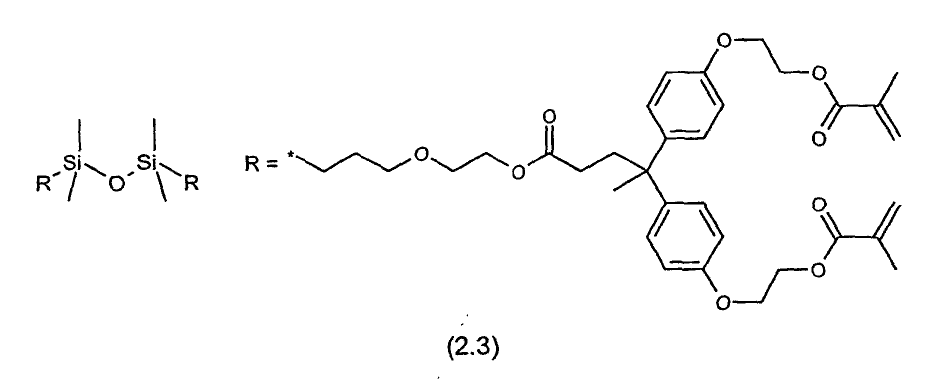 x = 2; y = 0; z = 0; K = Methyl, G-C[(Q-T-L)ₐA₃₋ₐ]; G = 4,7-Dioxa-8-oxo-1,10-decylen; A = Methyl; a = 2; Q = 1,4-Phenylen; T = 1,2-Ethandiol-diyl; L = Methacrylat. 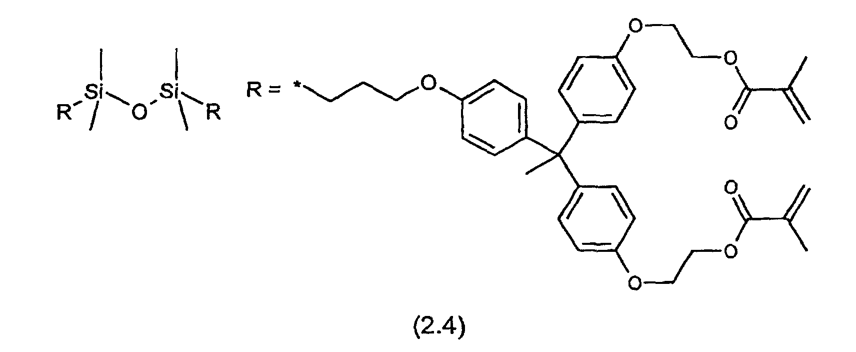 x = 2; y = 0; z = 0; K = Methyl, G-C[(Q-T-L)ₐA₃₋ₐ]; G = 4-Oxa-1,4-butylen-4-(1,4-phenylen; A = Methyl; a = 2; Q = 1,4-Phenylen; T = 1,2-Ethandiol-diyl; L = Methacrylat. 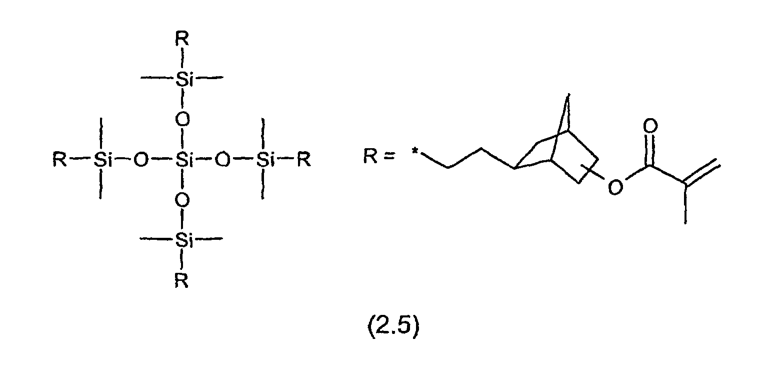 x = 4; y = 0; z = 1; K = Methyl, Q-L; Q = 2(3)-Oxyl-bicyclo[2.2.1]hept-5-(1,2-ethandiyl)-yl; L = Methacrylat; 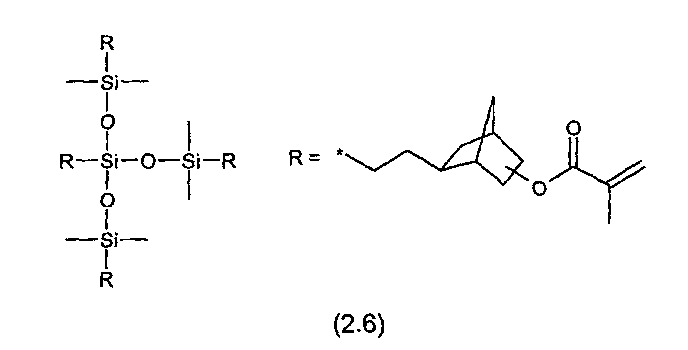 x = 3; y = 1; z = 0; K = Methyl, Q-L; Q = 2(3)-Oxyl-bicyclo[2.2.1]hept-5-(1,2-ethandiyl)-yl; L = Methacrylat; 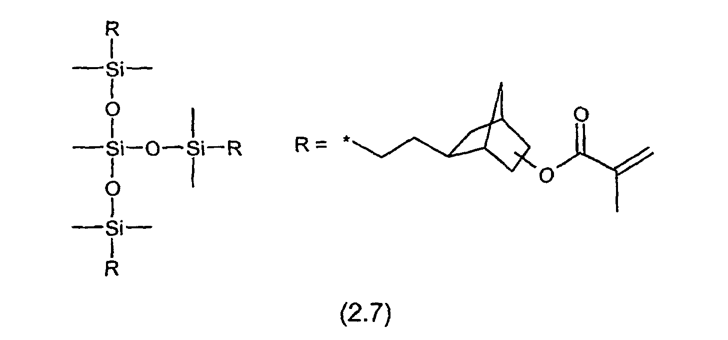 x = 3; y = 1; z = 0; K = Methyl, Q-L; Q = 2(3)-Oxyl-bicyclo[2.2.1]hept-5-(1,2-ethandiyl)-yl; L = Methacrylat; 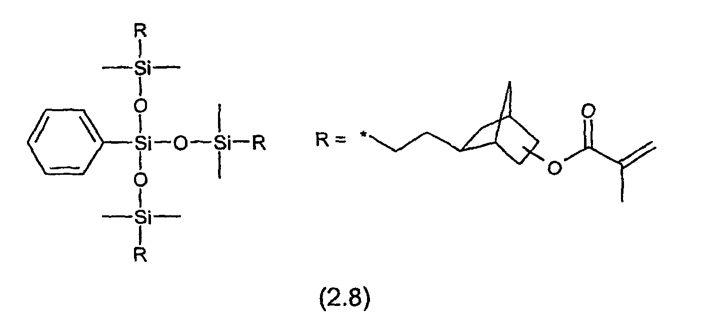 x = 3; y = 1; z = 0; K = Methyl, Phenyl, Q-L; Q = 2(3)-Oxyl-bicyclo[2.2.1]hept-5-(1,2-ethandiyl)-yl; L = Methacrylat; 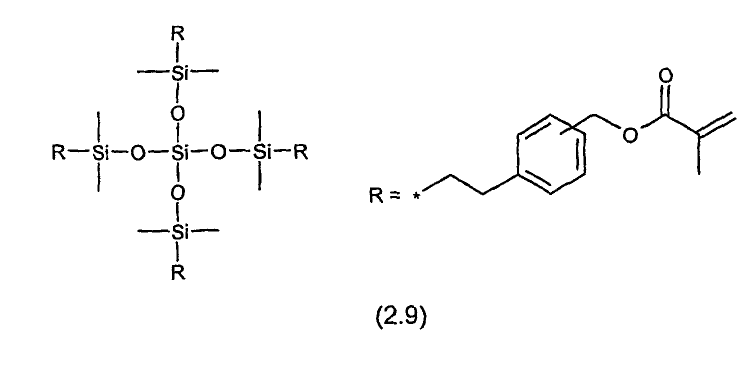 x = 4; y = 0; z = 1; K = Methyl, Q-L; Q = 1,2-Ethylen-(2-(4-methylenoxyl-1,4(3)-phenylen); L = Methacrylat; 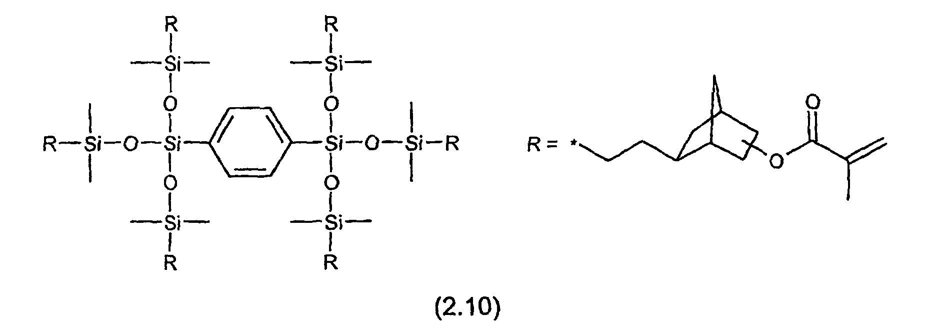 x = 4; y = 0; z = 1; K = Methyl, 1,4-Phenylen, Q-L; Q = 2(3)-Oxyl-bicyclo[2.2.1]hept-5-(1,2-ethandiyl)-yl; L = Methacrylat; 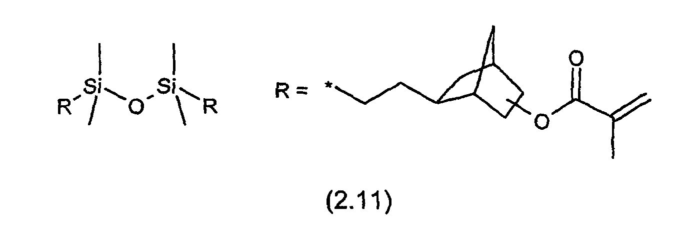 x = 2; y = 0; z = 0; K = Methyl, Q-L; Q = 2(3)-Oxyl-bicyclo[2.2.1]hept-5-(1,2-ethandiyl)-yl; L = Methacrylat; 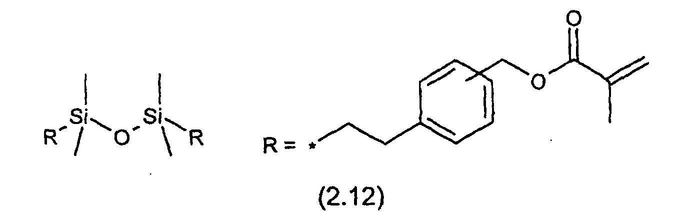 x = 2; y = 0; z = 0; K = Methyl, Q-L; Q = 1,2-Ethylen-(2-(4-methylenoxyl-1,4(3)-phenylen); L = Methacrylat;

Erfindungsgemäß sind auch aus den Monomeren gemäß den Formeln (1) und/oder (2) herstellbare härtbare Massen, enthaltend:
(K1) 0 bis 70, bevorzugt 0 bis 20 Gew.-% Monomere gemäß Formel (1),
(K2) 0 bis 70, bevorzugt 0 bis 20 Gew.-% Monomere gemäß Formel (2),
(K3) 0 bis 50, bevorzugt 3 bis 20 Gew.-% Co-Monomere,
(K4) 20 bis 90, bevorzugt 70 bis 85 Gew.-% Füllstoffe,
(K5) 0,001 bis 5, bevorzugt 0,1 bis 2 Gew.-% Initiatoren,
(K6) 0 bis 20, bevorzugt 0 bis 5 Gew.-% Hilfsstoffe,
mit der Maßgabe dass die Summe der Komponenten (K1) und (K2) mindestens 10 Gew.-%, bevorzugt mindestens 12 Gew.-% beträgt.

Co-Monomere gemäß Komponente (K3) sind mindestens einfach ethylenisch ungesättigt. Bevorzugt verwendete ethylenisch ungesättigte Co-Monomere sind Acrylate oder Methacrylate. Geeignet sind allgemein ein- und mehrfunktionelle (Meth)acrylatmonomere. Typische Vertreter dieser Verbindungsklasse (DE-A-43 28 960) sind Alkyl(meth)acrylate, einschließlich der Cycloalkyl(meth)acrylate, Aralkyl(meth)acrylate und 2-Hydroxyalkyl(meth)acrylate, beispielsweise Hydroxypropylmethacrylat, Hydroxyethylmethacrylat, Isobomylacrylat, Isobornylmethacrylat, Butylglycolmethacrylat, Acetylglykolmethacrylat, Triethylenglycoldimethacrylat, Polyethylenglycoldimethacrylat, 2-Phenylethylmethacrylat, 2-Ethylhexylmethacrylat, Cyclohexylmethacrylat, Laurylmethacrylat und Hexandioldi(meth)acrylat. Verwendet werden können auch langkettige Monomere auf der Basis von Bisphenol A und Glycidylmethacrylat, die aus der US-A-3 066 112 bekannt sind, oder deren durch Addition von Isocyanaten entstandenen Derivate. Geeignet sind auch Verbindungen des Typs Bisphenyl-Adiethyloxy(meth)acrylat und Bisphenol-A-dipropyloxy(meth)acrylat. Weiterhin Verwendung finden können die oligoethoxylierten und oligopropoxylierten Bisphenol-A-diacryl und -dimethacrylsäureester. Gut geeignet sind außerdem die in der DE-C-28 16 823 genannten Diacryl- und Dimethacrylsäureester des Bis(hydroxymethyl)-tricyclo[5.2.1.0^{2,6}]-decans und die Diacryl- und Dimethacrylsäureester der mit 1 bis 3 Ethylenoxid- und/oder Propylenoxideinheiten verlängerten Verbindungen des Bis(hydroxymethyl)-tricyclo[5.2.1.0^{2,6}]-decans.

Als Füllstoffe gemäß Komponente (K4) sind in der Regel anorganische Füllstoffe einsetzbar. Beispielhaft genannt seien Quarz, gemahlene Gläser, Kieselgele sowie pyrogene Kieselsäuren und Fällungskieselsäuren oder deren Granulate. Bevorzugt werden röntgenopake Füllstoffe, zumindest teilweise, mit eingesetzt. Diese können beispielsweise röntgenopake Gläser sein, also Gläser, welche beispielsweise Strontium, Barium oder Lanthan enthalten (z.B. nach US-A-3 971 754) oder ein Teil der Füllkörper besteht aus einem röntgenopaken Zusatz, wie beispielsweise Yttriumtrifluorid, Strontiumhexafluorozirkonat oder Fluoriden der Selten-Erdmetalle (z.B. nach EP-A-0 238 025). Zum besseren Einbau in die Polymermatrix ist es von Vorteil, die anorganischen Füllstoffe zu hydrophobieren. Übliche Hydrophobierungsmittel sind Silane, beispielsweise Trimethoxymethacryloyloxypropylsilan oder Trimethoxyglycidylsilan.

Die Füllkörper haben vorzugsweise eine mittlere Komverteilung <20 µm, insbesondere <5 µm, sowie eine obere Komgrenze von 150 µm, vorzugsweise 70 µm und insbesondere 25 µm.

Besonders bevorzugt werden Gemische von 5 bis 25 Gew.-% Füllstoffe mit einer mittleren Komgröße von 0,02 bis 0,06 µm und 65 bis 85 Gew.-% Füllstoffe mit einer mittleren Korngröße von 1 bis 5 µm verwendet.

Als Initiatoren gemäß Komponente (K5) werden solche Systeme verwendet, die in einem geeigneten Zeitraum Radikale zu bilden vermögen. Bei einkomponentigen Massen werden hierfür Photoinitiatoren eingesetzt, die durch Bestrahlung mit UVoder sichtbarem Licht die Polymerisationsreaktion auslösen können.

Vertreter solcher Photoinitiatoren sind beispielsweise Benzoinalkylether, Benzilketale, Acylphosphinoxide oder aliphatische und aromatische 1,2-Diketonverbindungen, beispielsweise Campherchinon, wobei die Lichtpolymerisation durch Zusatz von Aktivatoren, wie tertiären Aminen oder organischen Phosphiten, in an sich bekannter Weise beschleunigt werden kann.

Geeignete Initiatorsysteme zur Auslösung der Polymerisation über einen Redox-Mechanismus sind beispielsweise die Systeme Peroxid/Amin oder Peroxid/Barbitursäurederivate u. dergl. Bei Verwendung solcher Initiatorsysteme ist es zweckmäßig, einen Initiator (z.B. Peroxid) und eine Katalysatorkomponente (z.B. Amin) getrennt bereitzuhalten. Die beiden Komponenten werden dann kurz vor ihrer Anwendung miteinander homogen vermischt.

Geeignete Hilfsstoffe gemäß Komponente (K6) können beispielsweise üblicherweise auf dem Dentalgebiet eingesetzte Stabilisatoren, Pigmente oder Verdünnungsmittel sein.

Die Herstellung der hier offenbarten Massen erfolgt vorzugsweise so, dass die flüssigen Bestandteile miteinander gemischt werden, die Initiatoren, sofern sie nicht flüssig sind, darin durch Rühren eingebracht werden und anschließend die Füllstoffe zugegeben werden. Eine gute Komogenisierung kann durch Kneten erreicht werden.

Zweikomponentige Zubereitungen, deren Aushärtung durch Redox-Mechanismen erfolgt, werden so formuliert, dass die wesentlichen Bestandteile des Redoxlnitiierungssystems getrennt in je einem Teil der zweikomponentigen Zubereitung eingebracht werden. Die Aufteilung der Bestandteile der Gesamtzubereitung richtet sich nach den jeweiligen Lagerbeständigkeiten und dem angestrebten Mischungsverhältnis.

Die polymerisierbaren Massen zeichnen sich durch einen hohen Füllstoffanteil und damit verbundener hoher Festigkeit bei gleichzeitig guter Verarbeitbarkeit aus.

Die erfindungsgemäßen Massen eignen sich insbesondere als Werkstoffe für dentale Zwecke, beispielsweise zur Herstellung von Kunststoffzähnen oder Provisorien, als Beschichtungsmittel, zum Verkleben von Substraten sowie als dentale Füllungsmaterialien.

Die erfindungsgemäßen Massen werden üblicherweise in Behältnisse, wie Schlauchbeutel, Ein- oder Mehrkammerkartuschen oder Kapseln und andere Applikationseinheiten, beispielsweise Blisterverpackungen oder Spritzen, wie aus dem dentalen Bereich bekannt, eingebracht.

Die Erfindung wird nachfolgend durch Beispiele näher beschrieben, ohne dass sie durch diese begrenzt werden soll.

### Beispiele

### Herstellungsbeispiel 1

### Herstellung von 1,3,5,7-Tetrakis[2(3)-methacryloyl-bicyclo[2.2.1]heptan-5-(1,3-propandiyl]-1,3,5,7-tetramethyl-cyclotetrasiloxan (1.1)

10 g (41,6 mMol) 1,3,5,7-Tetramethyl-cyclotetrasiloxan werden in 50 ml Toluol gelöst und mit 34.3 g (166 mMol) 5-Vinyl-2(3)-norbornanyl-methacrylat (hergestellt gemäß US-3,927,116) und mit Karstedt-Katalysator (3 bis 3,5 % Pt, 200 ppm Pt, ABCR) 24 Stunden gerührt. Mittels IR wird der Umsatz geprüft und bei noch vorhandener Si-H-Bande bei etwa 2100 cm⁻¹ bis zum Verschwinden der Bande nachgerührt. Nach üblicher Aufarbeitung und Produktisolierung erhält man 45,2 g (93 %) eines hellgelben viskosen Öls. Viskosität η (23°C) = 70 Pa*s, n_{D}²⁰ = 1,496.

### Herstellungsbeispiel 2

### Herstellung von 1,3,5,7-Tetrakis[4(3)-methacryloylmethylen-phenyl-1-(1,2-ethandiyl]-1,3,5,7-tetramethyl-cyclotetrasiloxan (1.3)

10 g (41,6 mMol) 1,3,5,7-Tetramethyl-cyclotetrasiloxan werden in 50 ml Toluol gelöst und mit 33.7 g (166 mMol) 4(3)-Vinylbenzyl-methacrylat (hergestellt gemäß Beispiel 1 von EP-A-0 381 005) und mit Karstedt-Katalysator (3 bis 3,5 % Pt, 200 ppm Pt, Fa. ABCR) 24 Stunden gerührt. Mittels IR wird der Umsatz geprüft und bei noch vorhandener Si-H-Bande bei etwa 2100 cm⁻¹ bis zum Verschwinden der Bande nachgerührt. Nach üblicher Aufarbeitung und Produktisolierung erhält man 45,2 g (93 %) eines hellgelben viskosen Öls. Viskosität η (23°C) = 45 Pa*s, n_{D}²⁰ = 1,528.

### Herstellungsbeispiel 3

### Herstellung von 1,5-Bis[2(3)-methacryloyl-bicyclo[2.2.1]heptan-5-(1,2-ethandiyl]-1,1,5,5-tetramethyl-3,3-bis-[2-(2(3)-methacryloyl-bicyclo[2.2.1]heptan-5-(1,2-ethandiyl)-2,2-dimethylsiloxy]-trisiloxan (2.5)

10 g (30,4 mMol) Tetrakis-dimethylsiloxy-silan werden in 50 ml Toluol gelöst und mit 25,1 g (122 mMol) 5-Vinyl-2(3)-norbornanyl-methacrylat (hergestellt gemäß Beispiel XIV von US-3,927,116) und mit Karstedt-Katalysator (3 bis 3,5 % Pt, 200 ppm Pt, Fa. ABCR) 24 Stunden gerührt. Mittels IR wird der Umsatz geprüft und bei noch vorhandener Si-H-Bande bei etwa 2100 cm⁻¹ bis zum Verschwinden der Bande nachgerührt. Nach üblicher Aufarbeitung und Produktisolierung erhält man 45,2 g (93 %) eines hellgelben viskosen Öls. Viskosität η (23°C) = 12 Pa*s, n_{D}²⁰ = 1,492.

### Herstellungsbeispiel 4

### Herstellung von 1,4-Bis[2-(1,3,5,7-tetramethyl-1,3,5,7-(2(3)-methacryloylbicyclo[2.2.1]heptan-5-(1,2-ethandiyl)-cyclotetrasiloxanyl)-1,2-ethandiyl]-benzol (1.8)

625 g (2,6 Mol) 1,3,5,7-Tetramethyl-cyclotetrasiloxan werden mit 500 ml Toluol und 0,8 g Platin auf Aktivkohle bei Rückfluss vorgelegt. Dazu dotiert man 169 g Divinylbenzol (1,3 Mol; 80 %ig, technisch, Aldrich). Nach Verschwinden der vinylischen Protonen im ¹H-NMR arbeitet man in gewohnter Weise auf. Man erhält 587 g (92 % d. Th.) eines farblosen Öls mit der Viskosität η (23°C) = 0,3 Pa*s und einem Brechungsindex von 1,471.

21,7 g (0,105 Mol) 5-Vinyl-2(3)-norbomanyl-methacrylat und 50 ml Toluol werden mit Karstedt-Katalysator (3 bis 3,5 % Pt, 200 ppm Pt, ABCR) versetzt. Man tropft bei 50 °C 11,3 g des im vorherigen Absatz hergestellten Öls zu und rührt 24 Stunden. Mittels IR wird der Umsatz geprüft und bei noch vorhandener Si-H-Bande bei etwa 2100 cm⁻¹ bis zum Verschwinden der Bande nachgerührt. Nach üblicher Aufarbeitung und Produktisolierung erhält man 29,0 g (88 %) eines hellgelben viskosen Öls. Viskosität η (23°C) = 78 Pa*s, n_{D}²⁰ = 1,480.

### Herstellungsbeispiel 5

### Umsetzung eines SiH-Cyclengemisches mit einem Gemisch von Allyloxyethylmethacrylat und 5-Vinyl-2(3)-norbomanyl-methacrylat

10 g (166 mMol bezogen auf SiH) eines SiH-Cyclengemisches (40 % D^{H}₄, 45 % D^{H}₅, Rest höhere Ringe) werden in 50 ml Toluol gelöst und mit einem Gemisch von 17,2 g (84 mMol) 5-Vinyl-2(3)-norbomanyl-methacrylat (hergestellt gemäß US 3,927,116), 14,3 g (84 mMol) Allyloxyethylmethacrylat und Hexachlorplatinsäure (gelöst in i-Propanol) 24 Stunden gerührt. Mittels IR wird der Umsatz geprüft und bei noch vorhandener Si-H-Bande bei etwa 2100 cm⁻¹ bis zum Verschwinden der Bande nachgerührt. Nach üblicher Aufarbeitung und Produktisolierung erhält man 40 g (96 %) eines hellgelben viskosen Öls. Viskosität 2 Pa*s, n_{D}²⁰ = 1,486.

### Anwendungsbeispiele, Vergleichsbeispiel

In einem 100 ml Laborkneter wurden die pastösen Zubereitungen gemäß den Anwendungsbeispielen und dem Vergleichsbeispiel hergestellt, deren Zusammensetzungen in Tabelle 1 beschrieben sind.

Die Zubereitungen wurden hinsichtlich Druck- und Biegefestigkeit sowie Elastizitätsmodul gemäß DIN ISO 4049 charakterisiert.

Die Herstellung der Prüfkörper erfolgte durch Bestrahlung der in Formen eingebrachten Massen über einen Zeitraum von 40 Sekunden mit dem Lichtgerät Elipar® II der Firma ESPE Dental AG, Deutschland.

Die Prüfkörper wurden nach Entformung für einen Zeitraum von 24 Stunden in deionisiertes Wasser bei 36 °C eingelagert und danach die mechanischen Eigenschaften ermittelt.

Durch Bestimmung der Dichten der pastösen Zubereitungen und der ausgehärteten Massen nach der Archimedes'schen Auftriebsmethode wurde der bei der radikalischen Polymerisation auftretende Volumenschrumpf ermittelt.

Die Bestimmung der Opazität erfolgt mittels Probenkörper mit einer definierten Höhe von 3,5 (+/- 0,05) mm. Diese werden hergestellt, indem man das zu prüfende Material in entsprechend hohe Ringe bündig und blasenfrei einfüllt und zwischen planen glasklaren Matrizen je 40s mittels eines Lichtgerätes (Elipar® II, Fa. ESPE) im Kontakt belichtet. Die entformten Probekörper werden danach nochmals 15 min unter Vakuum in einem Lichtgerät (Visio® beta, Fa. ESPE) nachvergütet. Die Opazität wird dann mit dem Farbmessgerät "HunterLab LabScan Spectralcolorimeter" der Firma Hunter Lab Associates Laboratory, Inc., USA (Software SpecWare Software Version 1.10) gemessen und in %-Werten vom Gerät ausgegeben.

Eine Zusammenstellung der an den ausgehärteten Zubereitungen gemäß den Anwendungsbeispielen bzw. dem Vergleichsbeispiel ermittelten Eigenschaftswerten ist in Tabelle 2 dargestellt.

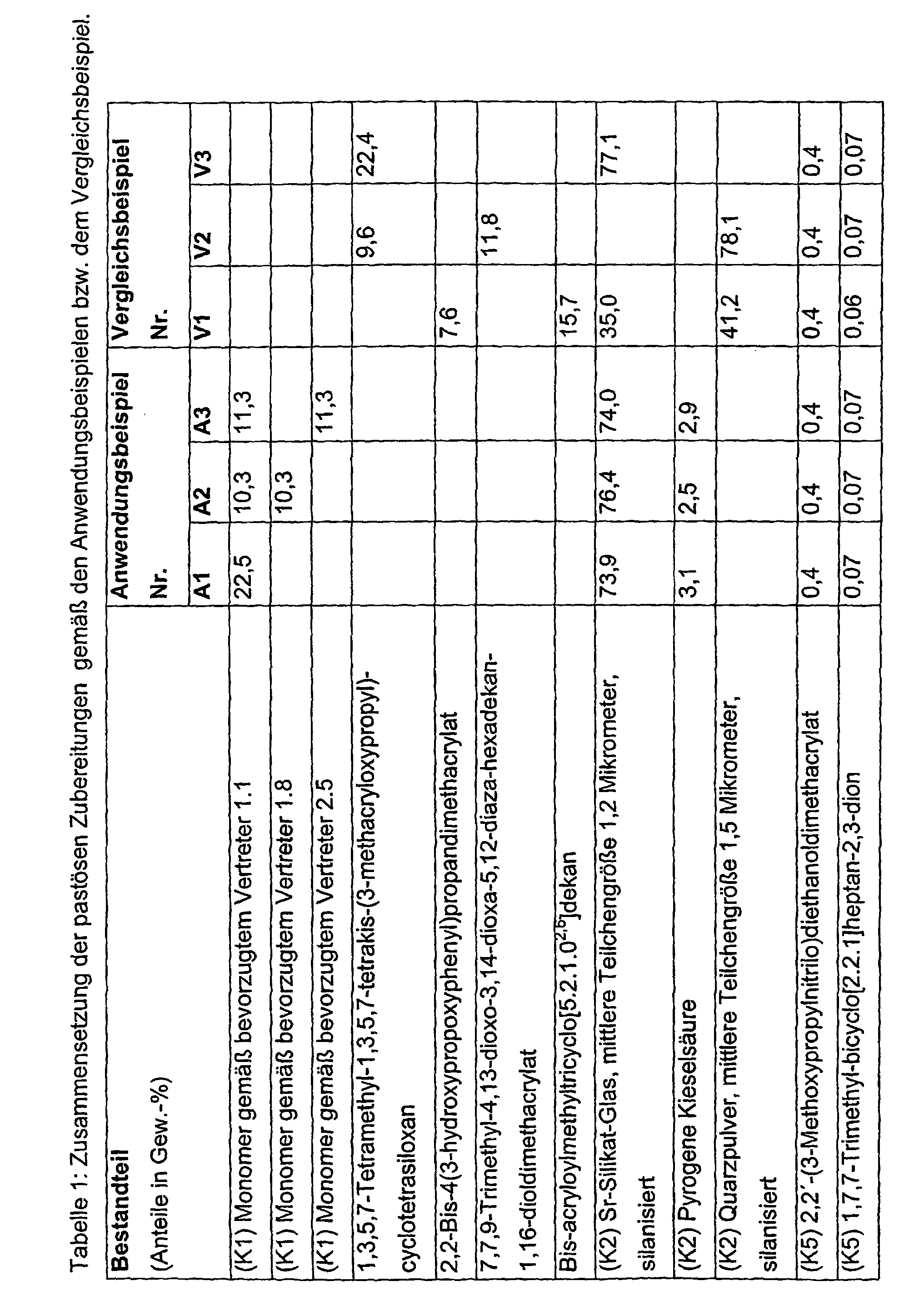

**Tabelle 2:**

| Zusammensetzung der Eigenschaftswerte, ermittelt an den ausgehärteten Zubereitungen gemäß den Anwendungsbeispielen bzw. dem Vergleichsbeispiel. | | | | | | |
|---|---|---|---|---|---|---|
| **Eigenschaft** | **Anwendungsbeispiel Nr.** | | | **Vergleichsbeispiel Nr.** | | |
| | **A1** | **A2** | **A3** | **V1** | **V2** | **V3** |
| Druckfestigkeit [MPa] | 380 | 373 | 405 | 411 | 416 | 345 |
| Biegefestigkeit [MPa] | 109 | 99 | 115 | 117 | 97 | 92 |
| Elastizitätsmodul [MPa] | 9678 | 8952 | 10025 | 8120 | 7348 | 4569 |
| Volumenschrumpf [%] | 1,70 | 2,35 | 2,32 | 3,67 | 2,81 | 2,67 |
| Opazität in [%] | 84 | 80 | 80 | 82 | 96 | 98 |

Die mit den aus den erfindungsgemäßen Monomeren hergestellten Dentalmassen zeigen eine Opazität von annähernd 85%, die den visuellen Eindruck natürlicher Zahnsubstanz ermöglicht. Die Vergleichsbeispiele sind entweder deutlich opaker, wodurch eine ungenügende ästhetische Wirkung des Zahnersatzmaterials erhalten wird, oder weisen ein Gehalt niedermolekularer Monomere auf und entsprechen daher nicht den toxikologischen Anforderungen.

Mit den aus den erfindungsgemäßen hochmolekularen Monomeren hergestellten Dentalmassen können bei optimaler Opazität hervorragende physikalische Werte erzielt werden.

Insbesondere der Volumenschrumpf ist äußerst gering und die Dentalmassen sind daher für die dauerhafte restaurative Versorgung eines Patienten gut geeignet.

## Patentansprüche

1. Monomere gemäß folgender Formel: 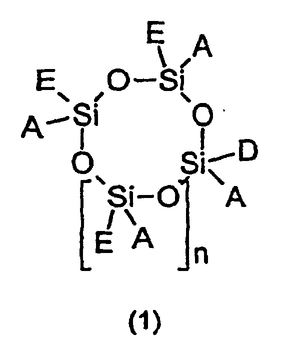 in welcher bedeuten:
n = eine ganze Zahl von 0 bis 10;
A = H oder lineare oder verzweigte C₁- bis C₁₅-Alk(en)yl oder C₃- bis C₁₅-Cycloalk(en)yl oder C₆- bis C₁₂-Aryl oder C₈-C₁₈-Alkaryl,
wobei aus den genannten Resten jeweils ein oder mehrere C-Atome durch O, C=O, O(C=4), SiR₂ und/oder NR ersetzt sein können, wobei R ein aliphatischer Rest mit 1 bis 7 C-Atomen ist, bei dem ein oder mehrere C-Atome durch O, C=O und/oder O(C=O) ersetzt sein können;
D = E oder ein Kohlenwasserstoff-Gerüst, das 2 bis 10, bevorzugt 2 bis 5 Cyclosiloxanreste verknüpft, wobei das Gerüst linear oder verzweigt oder cyclisch oder polycyclisch ist und 2 bis 50 C-Atome und zusätzlich 0 bis 30 andere Atome aus der Gruppe O, N, S, P, Cl, F, Br, I enthält und an welchem 1 bis 9 der oben definierten Cyclosiloxanreste gemäß Formel (1), abzüglich D, hängen;
E = A oder eine polymerisierbare Gruppe entnommen aus der Gruppe G-C[(Q-T-L)ₐ(A)₃₋ₐ] und/oder Q-L und gegebenenfalls zusätzlich X-T-L, wobei bis zu 50%, bevorzugt unter 25% bis 0% der Gruppen E in einem durchschnittlichen Molekül A entsprechen dürfen, mit der Maßgabe, dass bei Molekülen mit nur einem Siloxanring unter 25% bis 0% der Gruppen E in einem durchschnittlichen Molekül A entsprechen dürfen, und mit der Maßgabe, dass mindestens eine Gruppe G-C[(Q-T-L)ₐ(A)₃₋ₐ] und/oder Q-L im Molekül enthalten sein muss;
G = lineare, verzweigte oder cyclische C₁- bis C₂₅-Alk(en)ylen, Arylen, Alkarylen, Arylalkylen, wobei 0 bis 5 C-Atome ersetzt sein können durch einen Vertreter der Gruppe O, S, N-A, C(O), C(O)O, OC(O), C(O)N, NC(O), OC(O)O, NC(O)O, OC(O)N, NC(O)N;
X = C₁- bis C₁₀-Alk(en)ylen;
Q = ein in der Kette ein aromatisches oder polycyclisches Ringsystem enthaltender Rest, mit 5 bis 20 C-Atomen, der zusätzlich unabhängig voneinander 0 bis 5 Heteroatome aus der Gruppe O, N-A, S im Ringsystem aufweist;
T = O, N-A oder ein zwei- oder mehrwertiger linearer, verzweigter oder cyclischer Alkohol-, Amin- oder Aminoalkohol-Rest mit 2 bis 10 C-Atomen;
L = eine Acrylat- oder Methacrylatgruppe;
a = 2,3,
wobei die Monomeren eine (Meth)acrylatfunktionalität von über 2 aufweisen.

2. Monomere gemäß folgender Formel: 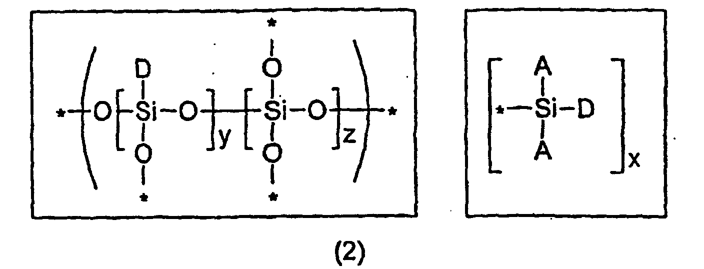 in welcher bedeuten:
x ≤ 2 + y + 2z;
y = 0, 1, 2, 3, 4, 5, 6, 7, 8;
z = 0, 1, 2, 3, 4, 5, 6, 7, 8;
und A, D die Bedeutung wie in Anspruch 1 angegeben haben; mit der Maßgabe, dass mindestens drei der Reste D in einem Molekül die Bedeutung von G-C[(Q-T-L)ₐ(A)₃₋ₐ] oder Q-L oder X-T-L haben müssen, und mit der Maßgabe, dass bei y = z = 0 mindestens ein Rest D die Bedeutung von G-C[(Q-T-L)ₐ(A)₃₋ₐ] hat , wobei die Monomeren eine (Meth)acrylatfunktionalität von über 2 aufweisen.

3. Verwendung der Monomere gemäß einem der vorangehenden Ansprüche zur Herstellung von härtbaren Massen.

4. Zusammensetzungen, enthaltend
(K1) 0 bis 70 Monomere gemäß Anspruch 1,
(K2) 0 bis 70 Monomere gemäß Anspruch 2,
(K3) 0 bis 50 Co-Monomere,
(K4) 20 bis 90 Gew.-% Füllstoffe,
(K5) 0,001 bis 5 Gew.-% Initiatoren,
(K6) 0 bis 20 Hilfsstoffe,
mit der Maßgabe dass die Summe der Komponenten (K1) und (K2) mindestens 10 Gew.-% beträgt.

5. Zusammensetzungen nach Anspruch 4 mit einer Opazität von 80 bis 90%.

6. Verwendung der Zusammensetzungen gemäß einem der Ansprüche 4 oder 5 zur Herstellung von härtbaren Massen, insbesondere von Dentalmassen.

7. Verwendung der Monomeren gemäß Anspruch 1 und/oder 2 oder der Zusammensetzungen gemäß einem der Ansprüche 4 oder 5 zur Herstellung von Dentalmassen mit einer Opazität, die geeignet ist, den visuellen Eindruck natürlicher Zahnsubstanz zu gewährleisten.

8. Behältnis, enthaltend mindestens eine Masse umfassend Monomere gemäß einem der Ansprüche 1 oder 2.

9. Behältnis, enthaltend mindestens eine Zusammensetzung gemäß einem der Anspruch 4 oder 5.

10. Verwendung von cyclischen oder linearen Siloxanen, enthaltend mindestens eine Seitenkette G-C[(Q-T-L)ₐ(A)₃₋ₐ] und/oder Q-L, wie sie in Anspruch 1 beschrieben ist, zur Herstellung von härtbaren Massen mit einer Opazität von 80 bis 90%.

## Claims

1. Monomers according to the following formula: 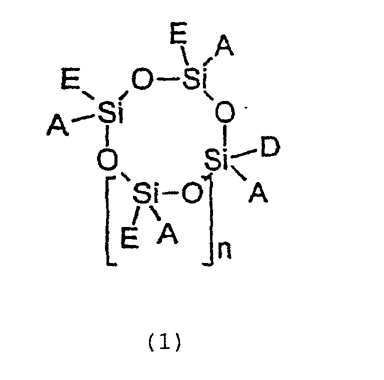 in which:
n = an integer from 0 to 10;
A = H or linear or branched C₁- to C₁₅-alk(en)yl or C₃- to C₁₅-cycloalk(en)yl or C₆- to C₁₂-aryl or C₈-C₁₈-alkaryl,
where, from the radicals mentioned, one or more C atoms can in each case be replaced by O, C=O, O(C=O), SiR₂ and/or NR, where R is an aliphatic radical having 1 to 7 C atoms, in which one or more C atoms can be replaced by O, C=O and/or O(C=O);
D = E or a hydrocarbon structure which links 2 to 10, preferably 2 to 5, cyclosiloxane radicals, the structure being linear or branched or cyclic or polycyclic and containing 2 to 50 C atoms and additionally 0 to 30 other atoms from the group consisting of O, N, S, P, Cl, F, Br, I and to which are attached 1 to 9 of the above-defined cyclosiloxane radicals according to Formula (1), minus D;
E = A or a polymerizable group taken from the group consisting of G-C[(Q-T-L)ₐ(A)₃₋ₐ] and/or Q-L and optionally additionally X-T-L, where up to 50%, preferably below 25% to 0%, of the groups E in an average molecule A must correspond, with the proviso that in molecules having only one siloxane ring below 25% to 0% of the groups E in an average molecule A must correspond, and with the proviso that at least one group G-C[(Q-T-L)ₐ(A)₃₋ₐ] and/or Q-L must be present in the molecule;
G = linear, branched or cyclic C₁- to C₂₅-alk(en)ylene, arylene, alkarylene, arylalkylene, where 0 to 5 C atoms can be replaced by a representative from the group consisting of O, S, N-A, C(O), C(O)O, OC(O), C(O)N, NC(O), OC(O)O, NC(O)O, OC(O)N, NC(O)N;
X = C₁- to C₁₀-alk(en)ylene;
Q = a radical containing an aromatic or polycyclic ring system in the chain, having 5 to 20 C atoms, which additionally independently of one another has 0 to 5 heteroatoms from the group consisting of O, N-A, S in the ring system;
T = O, N-A or a di- or polyhydric linear, branched or cyclic alcohol, amine or aminoalcohol radical having 2 to 10 C atoms;
L = an acrylate group or methacrylate group;
a = 2, 3,
where the monomers have a (meth)acrylate functionality of over 2.

2. Monomers according to the following formula: 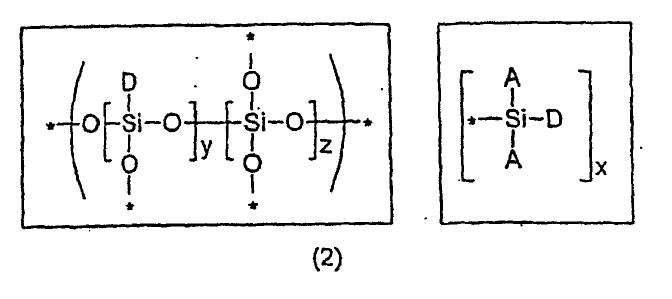 in which:
x ≤ 2 + y + 2z;
y = 0, 1, 2, 3, 4, 5, 6, 7, 8;
z = 0, 1, 2, 3, 4, 5, 6, 7, 8;
and A and D have the meaning as indicated in Claim 1; with the proviso that at least three of the radicals D in a molecule must have the meaning of G-C[(Q-T-L)ₐ(A)₃₋ₐ] or Q-L or X-T-L, and with the proviso that if y = z = 0 at least one radical D has the meaning of G-C[(Q-T-L)ₐ(A)₃₋ₐ], where the monomers have a (meth)acrylate functionality of over 2.

3. Use of the monomers according to one of the preceding claims for the production of curable compositions.

4. Compositions containing
(K1) 0 to 70 monomers according to Claim 1,
(K2) 0 to 70 monomers according to Claim 2,
(K3) 0 to 50 comonomers,
(K4) 20 to 90% by weight of fillers,
(K5) 0.001 to 5% by weight of initiators,
(K6) 0 to 20 excipients,
with the proviso that the sum of the components (K1) and (K2) is at least 10% by weight.

5. Compositions according to Claim 4 having an opacity of 80 to 90%.

6. Use of the compositions according to one of Claims 4 or 5 for the production of curable compositions, in particular of dental compositions.

7. Use of the monomers according to Claim 1 and/or 2 or of the compositions according to one of Claims 4 or 5 for the production of dental compositions having an opacity which is suitable for guaranteeing the visual impression of natural tooth substance.

8. Receptacle, containing at least one composition comprising monomers according to one of Claims 1 or 2.

9. Receptacle, containing at least one composition according to one of Claim 4 or 5.

10. Use of cyclic or linear siloxanes, containing at least one side chain G-C[(Q-T-L)ₐ(A)₃₋ₐ] and/or Q-L, as is described in Claim 1, for the production of curable compositions having an opacity of 80 to 90%.

## Revendications

1. Monomères de formule suivante : 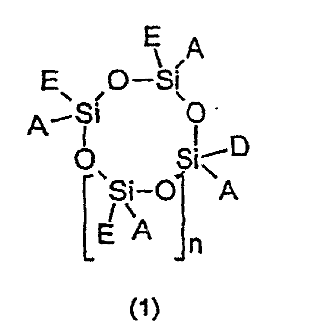 formule dans laquelle :
n représente un nombre entier allant de 0 à 10 ;
A représente H ou un groupe alkyle ou alcényle en C₁-C₁₅ linéaire ou ramifié, cycloalcényle en C₃-C₁₅, ou un groupe aryle en C₆-C₁₂ ou un groupe alkaryle en C₈-C₁₈,
un ou plusieurs atomes de carbone des radicaux cités pouvant être remplacés par O, C=O, O(C=O), SiR₂ et/ou NR, R étant un radical aliphatique ayant de 1 à 7 atomes de carbone, dans lequel un ou plusieurs atomes de carbone peuvent être remplacés par O, C=O et/ou O(C=O) ;
D représente E ou un squelette hydrocarboné qui relie 2 à 10, de préférence 2 à 5 restes cyclosiloxane, le squelette étant linéaire ou ramifié ou cyclique ou polycyclique et contenant de 2 à 50 atomes de carbone et additionnellement 0 à 30 autres atomes, choisis dans le groupe constitué par O, N, S, P, Cl, F, Br, I, et auquel sont accrochés 1 à 9 des restes cyclosiloxane de formule (1) définis plus haut, déduction faite de D ;
E représente A ou un groupe polymérisable choisi parmi G-C[(Q-T-L)ₐ(A)₃₋ₐ] et/ou Q-L et éventuellement en outre X-T-L, jusqu'à 50 %, de préférence moins de 25 % à 0 % du groupe E dans une molécule moyenne pouvant correspondre à A, avec la condition que dans les molécules ne comportant qu'un cycle siloxane, moins de 25 % à 0 % des groupes E dans une molécule moyenne peuvent correspondre à A, et avec la condition qu'au moins un groupe G-C[(Q-T-L)ₐ(A)₃₋ₐ] et/ou un groupe Q-L doit(doivent) être contenu(s) dans la molécule ;
G représente un groupe alkylène ou alcénylène, linéaire, ramifié ou cyclique, arylène, alkarylène, arylalkylène, en C₁-C₂₅, dans lequel 0 à 5 atomes de carbone peuvent être remplacés par un représentant de l'ensemble constitué par O, S, N-A, C(O), C(O)O, OC(O), C(O)N, NC(O), OC(O)O, NC(O)O, OC(O)N, NC(O)N ;
X représente un groupe alkylène ou alcénylène en C₁-C₁₀ ;
Q représente un radical contenant dans le chaîne un système cyclique aromatique ou polycyclique, ayant de 5 à 20 atomes de carbone, qui comporte en outre dans le système cyclique 0 à 5 hétéroatomes choisis indépendamment les uns des autres parmi O, NA, S ;
T représente O, N-A ou un radical alcool, amino ou aminoalcool di- ou polyvalent, linéaire, ramifié ou cyclique, ayant de 2 à 10 atomes de carbone;
L représente un groupe acrylate ou méthacrylate ;
a = 2, 3,
les monomères présentant une fonctionnalité (méth)acrylate supérieure à 2.

2. Monomères de formule suivante : 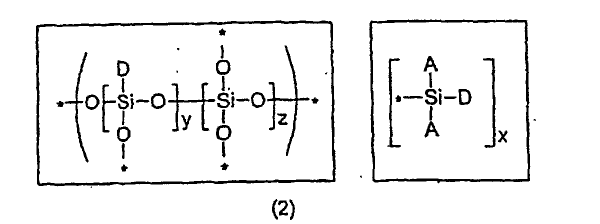 formules dans lesquelles :
x ≤ 2 + y + 2z ;
y = 0, 1, 2, 3, 4, 5, 6, 7, 8 ;
z = 0, 1, 2, 3, 4, 5, 6, 7, 8 ;
et A, D ont les significations indiquées dans la revendication 1, étant entendu qu'au moins trois des radicaux D dans une molécule doivent avoir la signification de G-C[(Q-T-L)ₐ(A)₃₋ₐ] ou Q-L ou X-T-L, et étant entendu que lorsque y = z = 0, au moins un radical D représente G-C[(Q-T-L)ₐ(A)₃₋ₐ], les monomères présentant une fonctionnalité (méth)acrylate supérieure à 2.

3. Utilisation des monomères selon l'une quelconque des revendications précédentes, pour la préparation de matières durcissables.

4. Compositions contenant
(K1) 0 à 70 % en poids de monomères selon la revendication 1,
(K2) 0 à 70 % en poids de monomères selon la revendication 2,
(K3) 0 à 50 % en poids de comonomères,
(K4) 20 à 90 % en poids de charges,
(K5) 0,001 à 5 % en poids d'amorceurs,
(K6) 0 à 20 % en poids d'adjuvants,
étant entendu que la somme des composants (K1) et (K2) est d'au moins 10 % en poids.

5. Compositions selon la revendication 4, ayant une opacité de 80 à 90 % .

6. Utilisation des compositions selon la revendication 4 ou 5, pour la préparation de matières durcissables, en particulier de matières dentaires.

7. Utilisation des monomères selon la revendication 1 et/ou la revendication 2 ou des compositions selon la revendication 4 ou 5, pour la préparation de matières dentaires ayant une opacité qui est appropriée à garantir l'impression visuelle de la substance dentaire naturelle.

8. Récipient contenant au moins une matière comprenant des monomères selon la revendication 1 ou 2.

9. Récipient contenant au moins une composition selon la revendication 4 ou 5.

10. Utilisation de siloxanes cycliques ou linéaires, contenant au moins une chaîne latérale G-C[(Q-T-L)ₐ(A)₃₋ₐ] et/ou Q-L, telle que décrite dans la revendication 1, pour la préparation de matières durcissables ayant une opacité de 80 à 90 %.
